# EUROPÄISCHE PATENTSCHRIFT

(11) **EP 0 776 179 B1**
(45) Veröffentlichungstag und Bekanntmachung des Hinweises auf die Patenterteilung: **04.03.1998**
(21) Anmeldenummer: 95928958.8
(22) Anmeldetag: 17.08.1995
(51) Int. Cl.: A61B 17/12

(54) **PUNKTIONSVERSCHLUSS**
PUNCTURE CLOSURE
TAMPON POUR ORIFICE DE PONCTION

(30) Priorität: 18.08.1994 DE 4429230
(43) Veröffentlichungstag der Anmeldung: 04.06.1997
(73) Patentinhaber: Harren, Ernst-Diethelm, 52146 Würselen (DE); Bangert, Christian, 52066 Aachen (DE)
(72) Erfinder: Harren, Ernst-Diethelm, 52146 Würselen (DE); Bangert, Christian, 52066 Aachen (DE)
(74) Vertreter: WALTHER, WALTHER & HINZ Patentanwälte
(86) Internationale Anmeldenummer: DE9501108
(87) Internationale Veröffentlichungsnummer: WO9605774

(56) Entgegenhaltungen:
- EP-A- 0 514 026
- EP-A- 0 554 602
- US-A- 3 954 109
- US-A- 5 234 459

## Beschreibung

Die Erfindung betrifft einen Punktionsverschluß zum Verschließen eines eine Punktionsöffnung aufweisenden Blutgefäßes, insbesondere einer Arterie, einer mit einer Arterie kurzgeschlossenen Vene, eines Shunts bzw. einer Prothese usw., in dem ein arterieller Druck herrscht, mit einer mit Überdruck beaufschlagbaren Druckkammer, die eine Öffnung zur Aufnahme eines Druckmediums aufweist und die im Bereich der Punktionsöffnung am Körper befestigbar ist, wobei der dem Körper zugewandte Teil der Druckkammer dehnbar ausgebildet ist.

Blutgefäße im Sinne der vorliegenden Erfindung sind sämtliche arteriellen Gefäße, d.h., sämtliche vom Herzen wegführenden Gefäße oder Gefäße in denen ein arterieller Druck (133,322-266,644 mbar) herrscht. Darunter fallen auch Venen, die mit einer Arterie kurzgeschlossen sind und an eine Arterie angeschlossene Prothesen (Interponate oder Shunts), denn bei allen diesen Blutgefäßen ergibt sich nach einen) medizinischen Eingriff das Problem des anschließenden Wundverschlusses.

Bei einer Vielzahl von invasiven Eingriffen am menschlichen und tierischen Körper ist es erforderlich, daß sich der Arzt Zugang zu einer Arterie oder einem arteriellen Gefäß verschafft. Zu diesen invasiven Eingriffen gehören beispielsweise Katheteruntersuchungen jeder Art, wie Arteriographien am Herzen, im Hirn usw. mittels eines in eine Arterie eingespritzten Röntgenkontrastmittels; Ballondilatationen oder Fräsungen der Arterien; Thrombektomien; Funktionsuntersuchungen des linken Herzens oder das Einbringen von Medikamenten in bestimmte Durchblutungsgebiete, etwa der Herzkranzgefäße zur Lyse.

Darüberhinaus sind auch Patienten der chronischen Hämodialyse betroffen, denn zur extrakorporalen Reinigung des Blutes ist ein von außen gut erreichbares arterialisiertes Gefäß operativ angelegt worden. Es handelt sich bei diesem Gefäß beispielsweise um ein eigenes, venöses Gefäß oder um einen eingesetzten Kunststoffschlauch, der mit der Arterie kurzgeschlossen wird (Shunt).

Nach jedem Blutreinigungsvorgang, also mehrmals pro Woche, ergibt sich auch hier das Problem, daß der punktierte Shunt sich nur schwer verschließen läßt.

Das Blut preßt sich mit einem Druck von 133,322 - 266,644 mbar durch die Punktionsöffnung der Gefäßwand ins Freie oder in das die Arterie umgebene Gewebe. Wegen des vergleichsweise hohen Druckes in der Arterie geht in kürzester Zeit eine große Menge Blut verloren und es kann zu einer unerwünschten Hämatombildung im umliegenden Gewebe führen.

Blutverlust und Hämatombildung sind unbedingt zu vermeiden. Dies geschieht derzeit dadurch, daß mit dein Finger ein adäquater Druck auf die Arterie oder das arterielle Gefäß ausgeübt wird.

Dieser Druck ist individuell zu dosieren und richtet sich nach dem herrschenden Blutdruck im Gefäß sowie nach der Tiefe und Konsistenz des darüberliegenden Gewebes. Er schwankt interindividuell (von Patient zu Patient) und intraindividuell (abhängig von Blutdruckschwankungen, vor allem von Blutdruckabfällen des einzelnen Patienten) und muß kontinuierlich überprüft und angepaßt werden.

Wenn der ausgeübte Druck zu schwach ist, kann die Hämatombildung mit mehreren Litern beachtliche Ausmaße und schwerwiegende Folgen annehmen. Beispielhaft seien hier einige der zu vermeidenden Folgen genannt: Hämodynamische Schwierigkeiten, d.h., Kreislaufprobleme wenn durch das restliche, intravasale Blutvolumen der Verlust nicht mehr kompensiert werden kann; Kompartmentsyndrome, d.h., Kompression von wichtigen Strukturen (Nerven, Shunt) durch das Hämatom (oder durch den Druck eines sich zusätzlich entwickelnden Ödems) mit der Folge der Zerstörung dieser Strukturen; lokale sterile Entzündungsreaktionen bei der Resorption und Organisation (Gewebeumbau) des Hämatoms; Infektion des Hämatoms; und möglicherweise die Notwendigkeit zur operativen Ausräumung des Hämatoms mit weiteren Risiken wie Infektion, Abzeß, sekundäre Wundheilung usw..

Andererseits darf der ausgeübte Druck aber nicht zu stark sein, da das Blutgefäß sonst vollständig kollabieren würde. Mögliche Folgen eines zu starken Druckes sind Durchblutungsstörungen der nachfolgenden Strukturen (z.B. eines Beines usw.) mit evtl. irreversiblem Verlust durch die ischämische Gewebeschädigung oder, vor allem bei Dialysepatienten, Shuntverschluß durch Thrombosebildung wegen Hämostase.

Wegen der Schwere der Komplikationen haben sich ungenaue Systeme des Punktionsverschlusses, z.B. Klemmen oder zirkuläre Staubinden usw. bisher nicht durchsetzen können. Gleiches gilt für die in der EP-0 554 602 und EP-0 514 026 vorgeschlagenen Punktionsverschlüsse. Bei diesen Punktionsverschlüssen wird eine Druckkammer mittels eines umlaufenden Bandes am Körper festgebunden und mit Druckluft beaufschlagt. Das Ausdehnen der Druckkammer erzeugt dann einen Druck auf das den Punktionsverschluß umgebende Gewebe. Hierdurch wird lediglich der manuell ausgeübte Druck durch einen Druckverband ersetzt, bei dem die oben genannten Probleme der genauen Dosierung und der Anpassung an die jeweilige Situation bestehen bleiben.

Deshalb wird der Druck nach wie vor mit einem Finger ausgeübt, denn durch den Tastsinn kann der Druck individuell und variabel dosiert werden.

Oftmals ist der Patient dazu leider nicht in der Lage. Eine Schwächung im Rahmen der Grunderkrankung, eine Sedierung wegen der erfolgten Untersuchung oder ein Mangel an Erfahrung machen es dem Patienten unmöglich die oben beschriebene Tätigkeit selbst durchzuführen.

Das bedeutet, daß das Pflegepersonal oder das ärztliche Personal diese Tätigkeit übernimmt und damit eine beträchtliche Zeit, nämlich zwischen 10 und 60 Minuten, blockiert ist.

Dies ist sowohl für das Pflegepersonal, als auch für das ärztliche Personal zeitraubend und für den Träger teuer und uneffektiv. Außerdem zeigen die oben beschriebenen Komplikationen, daß die praktizierte beste Lösung noch nicht gut genug ist.

Davon ausgehend liegt der Erfindung die Aufgabe zugrunde, einen Punktionsverschluß zu schaffen, der die Punktionsöffnung eines Blutgefäßes zuverlässig verschließt, ohne daß ein zu großer Blutverlust auftritt, ohne daß sich nennenswerte Hämatome bilden und ohne daß das Blutgefäß vollständig kollabiert.

Der Erfindung liegt die Erkenntnis zugrunde, daß das Blut solange aus der Öffnung des arteriellen Gefäßes herausfließen wird, bis sich im umliegenden Gewebe ein adäquater Gegendruck, d. h. ein dem Blutdruck entsprechender Druck, aufgebaut hat. Falls die Öffnung des arteriellen Gefäßes nicht manuell abgedrückt wird, fließt das Blut in das umliegende Gewebe und bewirkt in diesem die zur Stillung der Blutung erforderliche Druckerhöhung. Durch Aufnahme des zusätzlichen (Blut-) Volumens im Gewebe vergrößert sich das Gewebe lokal und drückt auf die Punktionsöffnung. Sobald der durch das Gewebe erzeugte Druck dem Blutdruck entspricht, herrscht an der Öffnung des arteriellen Gefäßes ein Druckgleichgewicht, das die Blutung zum Stillstand bringt.

Die oben beschriebene Aufgabe wird erfindungsgemäß dadurch gelöst, daß die Öffnung zur Aufnahme des Druckmediums über einem Einstichkanal der Punktionsöffnung angeordnet ist und daß als Druckmedium, das aus dem Blutgefäß herausströmende Blut eingesetzt ist.

Bei einem invasiven Eingriff, bei dem eine Arterie geöffnet wird, entsteht ein sogenannter Einstichkanal. Dieser Einstichkanal führt von der Punktionsöffnung des Blutgefäßes direkt durch das darüberliegende Gewebe und die Haut und ist an der Oberfläche des Körpers gut sichtbar. Wird z.B. mit einer Spritze etwas in die Arterie injiziert, so ist die durch die Kanüle gebildete Öffnung der Einstichkanal. Nachdem die Kanüle herausgezogen wurde, strömt das Blut durch die Punktionsöffnung der Arterie in diesen Einstichkanal und anschließend ins Freie. Wenn nun das hier ausströmende Blut in der Druckkammer des erfindungsgemäßen Punktionsverschlusses aufgefangen wird, so hat dies mehrere Vorteile:
1. Das Blut wird kontrolliert aufgefangen, was zu einer erhöhten Hygiene am Krankenbett führt.
2. Dieses Blut wird als Druckmedium eingesetzt, so daß sich innerhalb kürzester Zeit in der Druckkammer des Punktionsverschlusses der in der Arterie herrschende Druck ausbildet und auf das um den Einstichkanal herum befindliche Gewebe drückt. Damit erhöht sich gleichzeitig der Druck im Gewebe.
3. Das Abfließen des Blutes in das Gewebe wird zuverlässig verhindert und eine Hämatombildung wird ausgeschlossen.
4. Eine derart individuell an die Person und die jeweilige Situation angepaßte Druckregulierung im Punktionsverschluß führt zu dem Ergebnis, daß das Ausfließen des Blutes aus der Arterie in dem Moment gestoppt wird, in dem in der Druckkammer derselbe Druck wie in der Arterie anliegt. Da die Druckkammer nur ein sehr geringes Fassungsvermögen aufweist, ist der gewünschte Druck sehr schnell erreicht.
5. Der Patient erleidet keinen nennenswerten Blutverlust.

Nachdem die Blutung gestillt ist und das Blut geronnen ist, kann der Punktionsverschluß problemlos entfernt werden.

In einer besonders bevorzugten Ausführungsform des erfindungsgemäßen Punktionsverschlusses ist die Druckkammer aus einem dehnbaren, bis auf die Öffnung zur Aufnahme des Druckmediums geschlossenen Behälter gebildet.

Dies hat den Vorteil, daß sich die Druckkammer, nachdem sie mit Druck beaufschlagt wurde, ungehindert ausdehnen kann und den gewünschten Druck auf die Körperstelle, insbesondere auf das unter der Haut liegende Gewebe ausüben kann.

In einer anderen Ausführungsform ist der Behälter am Halteband befestigt, vorzugsweise festgeklebt. Dadurch bildet der Teil des dehnbaren Behälters, der am Halteband befestigt ist, zusammen mit dem Halteband eine nahezu starre Haltewand und der übrige Teil des Behälters bildet eine dehnbare Druckwand.

Dies hat den Vorteil, daß die Herstellung kostengünstig wird, da nur einfache, ebene Teile ohne Hinterschneidung zum Einsatz kommen.

In einer weiteren, bevorzugten Ausführungsform des erfindungsgemäßen Punktionsverschlusses ist der dem Körper zugewandte Teil der Druckkammer als eine dehnbare Druckwand und der dem Körper abgewandte Teil der Druckkammer als eine nahezu starre Haltewand ausgebildet.

In einer anderen, besonders bevorzugten Ausführungsform des erfindungsgemäßen Punktionsverschlusses ist die dehnbare Druckwand an einem am Halteband befestigten Trägerelement druckdicht befestigt, vorzugsweise festgeklebt.

In einer besonderen Ausführungsform ist die nahezu starre Haltewand durch das Halteband gebildet und die dehnbare Druckwand ist druckdicht, vorzugsweise geklebt an dem Halteband befestigt.

Dies hat den Vorteil, daß der Punktionsverschluß kostengünstig und mit einem niedrigen Materialaufwand hergestellt werden kann.

Die letztgenannten Ausführungsformen haben den Vorteil, daß die nahezu starre Haltewand eine Ausdehnung der Druckkammer vorn Körper weg verhindert, so daß der vorhandene Druck ausschließlich die Druckwand ausdehnt. Da aber die Druckwand dem Körper zugewandt ist, kann der vorhandene Druck vollständig zur Druckbildung auf das Gewebe genutzt werden. Dadurch kann die gewünschte Wirkung bereits mit einem geringen Volumen erreicht werden.

In einer anderen Ausführungsform ist das Halteband ein Gewebeband, ein Pflaster, ein Kunststoffband, Inzisionsfolie oder dergleichen. Mit einem derartigen Halteband kann der Punktionsverschluß kostengünstig hergestellt werden.

In einer weiteren, bevorzugten Ausführungsform des erfindungsgemäßen Punktionsverschlusses ist das Halteband luftdurchlässig.

Dies hat den Vorteil, daß die Haut im Bereich des Haltebandes noch atmen kann.

In einer weiteren Ausführungsform ist das Halteband auf der dem Körper zugewandten Seite zumindest teilweise mit Klebstoff versehen. Dadurch kann das Halteband und somit der Punktionsverschluß schnell und in einfacher Weise an der gewünschten Körperstelle aufgeklebt werden.

In einer bevorzugten Ausführungsform ist die dehnbare Druckwand oder der dehnbare Behälter aus Gummi, Latex, Silicon oder Kunststoff oder dergleichen hergestellt.

Dies hat den Vorteil, daß die Druckkammer ohne großen Kraftaufwand gedehnt werden kann und daß der vorhandene Druck vollständig zur Druckbildung auf das Gewebe genutzt werden kann.

In einer weiteren, bevorzugten Ausführungsform sind in der Druckkammer blutstillende Wirkstoffe, beispielsweise Blutgerinnungsmittel (Hämostatika), Desinfektionsmittel oder dergleichen angeordnet. Dadurch wird das Gerinnen des Blutes beschleunigt.

In einer anderen erfindungsgemäßen Ausführungsform ist die Druckkammer mit einem Füllstoff versehen, der aufquillt, sobald er mit dem Druckmedium in Kontakt kommt. Dazu wird vorzugsweise Mull, Watte oder ein aufquellender Kunststoff eingesetzt.

Dies hat den Vorteil, daß der zum Wundverschluß erforderliche Druck in der Druckkammer sehr viel schneller aufgebaut wird und der Blutverlust des Patienten weiter verringert werden kann.

In einer weiteren Ausführungsform ist die Aussparung in der Haltewand mit einem aufklebbaren Verschlußelement verschließbar. Dadurch wird die für die, bzw. von der Kanüle geschaffene Aussparung in der Haltewand zuverlässig und druckdicht verschlossen.

In weiteren Ausführungsformen ist am Verschlußelement ein Füllstoff oder ein Druckpolster angebracht.

Beide haben den Vorteil daß der in der Druckkammer erforderliche Druck sehr viel schneller erreicht wird und der Patient deutlich weniger Blut verliert.

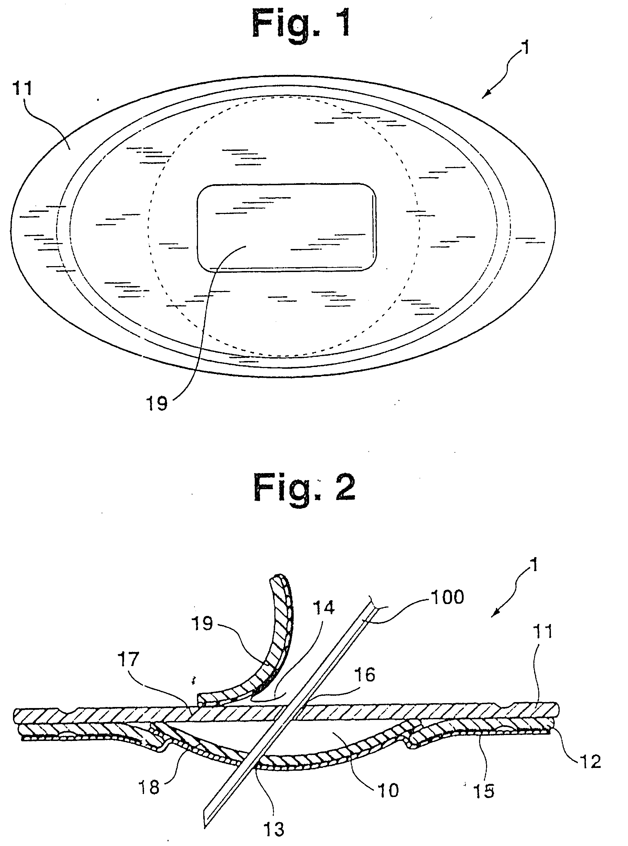

Weitere Vorteile der erfindungsgemäßen Vorrichtung ergeben sich aus der Beschreibung der einzelnen Ausführungsformen und der beigefügten Zeichnung. Ebenso können die vorstehend genannten und die noch weiter aufgeführten Merkmale erfindungsgemäß jeweils einzeln oder in beliebigen Kombinationen miteinander verwirklicht werden. Die erwähnten Ausführungsformen sind nicht als abschließende Aufzählung zu verstehen, sondern haben vielmehr beispielhaften Charakter. Ausführungsformen der Erfindung sind in den Figuren 1 bis 6 der Zeichnung dargestellt und werden im Folgenden näher erläutert.
- Fig. 1: zeigt eine Draufsicht auf eine erste Ausführungsform eines erfindungsgemäßen Punktionsverschlusses;
- Fig. 2: zeigt eine geschnitten dargestellte Seitenansicht des Punktionsverschlusses gemäß Fig. 1;
- Fig. 3: zeigt eine geschnitten dargestellte Seitenansicht einer zweiten Ausführungsform eines erfindungsgemäßen Punktionsverschlusses;
- Fig. 4: zeigt eine geschnitten dargestellte Seitenansicht einer dritten Ausführungsform eines erfindungsgemäßen Punktionsverschlusses;
- Fig. 5: zeigt eine geschnitten dargestellte Seitenansicht des Punktionsverschlusses gemäß Figuren 1 und 2, wobei die Druckkammer mit einem Füllstoff gefüllt ist;
- Fig. 6: zeigt eine geschnitten dargestellte Seitenansicht des Punktionsverschlusses gemäß Figuren 1 und 2, wobei ein zusätzliches Druckpolster vorgesehen ist;

Die einzelnen Figuren der Zeichnung zeigen den erfindungsgemäßen Gegenstand teilweise stark schematisiert und sind nicht maßstäblich zu verstehen. Hierbei sind die Gegenstunde der einzelnen Figuren teilweise stark überproportional vergrößert dargestellt, damit ihr Aufbau besser gezeigt werden kann.

Die **Figuren 1** und **2** zeigen eine erste Ausführungsform eines erfindungsgemäßen Punktionsverschlusses, wobei der gezeigte Punktionsverschluß **1** im wesentlichen oval ausgeführt ist. In anderen Ausführungsformen sind auch runde, dreieckige, viereckige oder andersförmige Punktionsverschlüsse denkbar.

Der Punktionsverschluß **1** setzt sich zusammen aus einem nahezu nicht dehnbaren Halteband **11**, einem Trägerelement **12**, einer dehnbaren Druckwand **18** und einem Verschlußelement **19**. Das Trägerelement **12** hat eine kreisrunde Öffnung, in die die dehnbare Druckwand **18** eingesetzt ist. Im Überlappungsbereich von Trägerelement **12** und Druckwand **18** sind beide miteinander verklebt, so daß eine druckdichte Verbindung entsteht, wobei die Druckwand **18** zwischen dem Trägerelement **12** und dem Halteband **11** angeordnet ist. Dabei bildet das Halteband **11** gleichzeitig eine Haltewand **17** der Druckkammer **10**.

Das Trägerelement **12** und das Halteband **11** sind durch verschweißen und/oder verkleben ebenfalls druckdicht miteinander verbunden. Das Trägerelement **12** und das Halteband **11** sind in dieser Ausführungsform aus demselben, nur wenig dehnbaren Material hergestellt.

In eine Öffnung **13** der dehnbaren Druckwand **18** und in eine Aussparung **16** der nahezu nicht dehnbaren Haltewand **17** ist eine Kanüle **100** eingesetzt, mit der der invasive Eingriff durchgeführt wird. Nach Abschluß des invasiven Eingriffs und nachdem die Kanüle **100** entfernt worden ist, kann die Aussparung **16** durch ein Verschlußelement **19** verschlossen werden. Dabei wird das Verschlußelement **19** auf das Halteband **11** aufgeklebt. Auf die Klebeschicht des Verschlußelementes **19** ist eine Schutzfolie **14** aufgebracht, die vor dem Ankleben des Verschlußelementes **19** entfernt werden muß.

Um den Punktionsverschluß **1** zuverlässig auf dem Körper zu befestigen, ist die gesamte Unterseite des Punktionsverschlusses **1**, d. h. die dem Körper zugewandte Seite, mit einem hautfreundlichen Klebstoff **15** versehen.

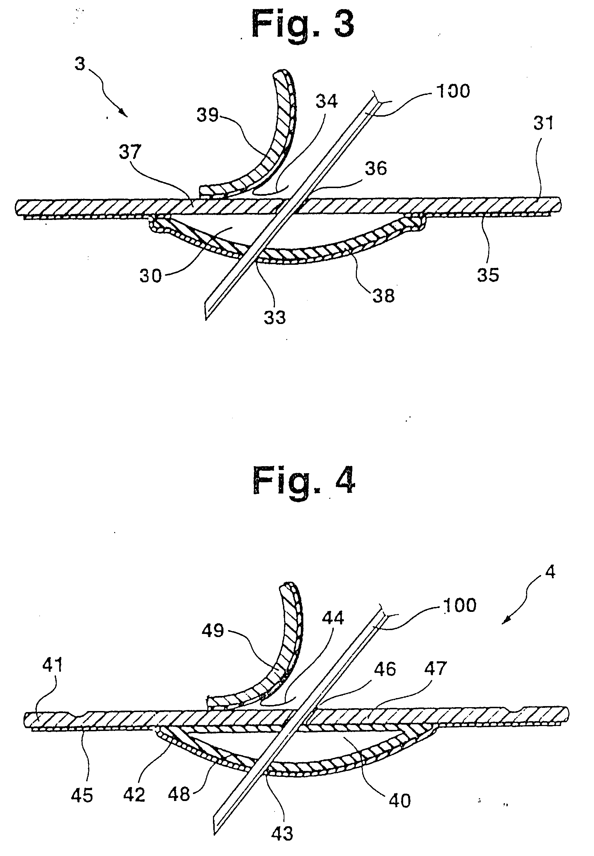

**Figur 3** zeigt eine zweite Ausführungsform **3** eines erfindungsgemäßen Punktionsverschlusses, bei dem eine dehnbare Druckwand **38** zur Bildung einer Druckkammer **30** auf ein Halteband **31** aufgeklebt ist. Dabei bildet das Halteband **31** gleichzeitig eine Haltewand **37** der Druckkammer **30**. In eine Öffnung **33** der dehnbaren Druckwand **38** und in eine Aussparung **36** der nahezu nicht dehnbaren Haltewand **37** ist eine Kanüle **100** eingesetzt, mit der der invasive Eingriff durchgeführt wird. Nach Abschluß des invasiven Eingriffs und nachdem die Kanüle **100** entfernt worden ist, kann die Aussparung **36** hier auch durch ein Verschlußelement **39** verschlossen werden. Dabei wird das Verschlußelement **39** ebenfalls auf das Halteband **31** aufgeklebt. Auf die Klebeschicht des Verschlußelementes **39** ist eine Schutzfolie **34** aufgebracht, die vor dem Ankleben des Verschlußelementes **39** entfernt werden muß.

Auch hier ist die dem Körper zugewandten Seite des Punktionsverschlusses **3** mit einem Klebstoff **35** versehen.

**Figur 4** zeigt eine dritte Ausführungsform **4** eines erfindungsgemäßen Punktionsverschlusses, die eine aus einem im wesentlichen geschlossenen Behälter **42** gebildete Druckkammer **40** aufweist. Hierbei ist der Behälter **42** an einem Halteband **41** festgeklebt, so daß der Bereich des Behälters **42**, der an dem Halteband **41** befestigt ist, eine Haltewand **47** bildet. Der übrige Bereich des dehnbaren Behälters **42** bildet eine dehnbare Druckwand **48**. In eine Aussparung **46** der Haltewand **47** und in eine Öffnung **43** der Druckwand **48** ist auch hier eine Kanüle **100** eingesetzt. Auch hier wird die Aussparung **46** von einem mit einer Schtutzfolie **44** versehenen Verschlußelement **49** verschlossen.

Auch hier ist die dem Körper zugewandten Seite des Punktionsverschlusses **4** mit einem Klebstoff **45** versehen.

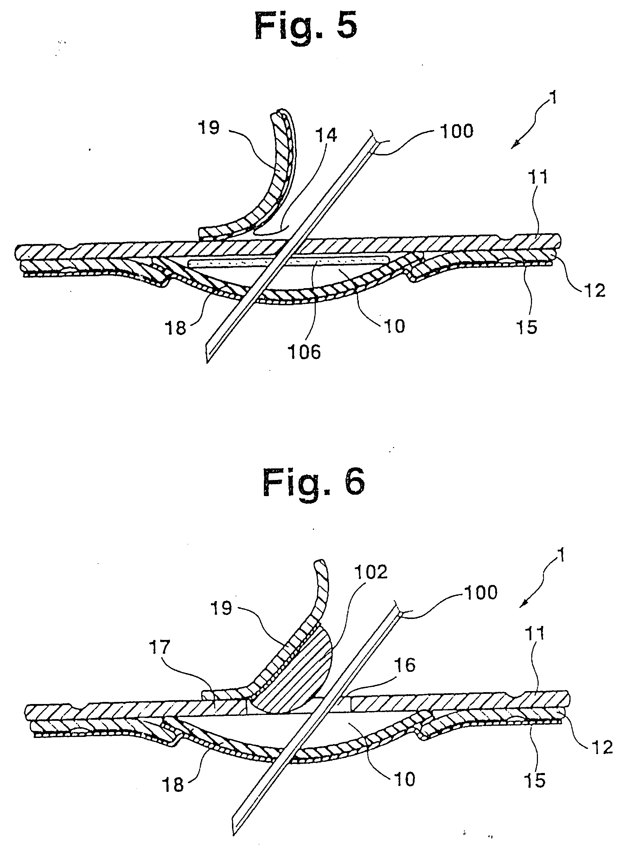

Die **Figuren 5** und **6** zeigen Weiterbildungen der ersten Ausführungsformen. Diese Weiterbildungen können auch auf andere Ausführungsformen angewendet werden, ohne den Gegenstand der Erfindung zu verlassen.

Die Druckkammer **10** des in **Figur 5** dargestellten Punktionsverschlusses **1** enthält einen Füllstoff **106**, der dazu dient, den in der Druckkammer **10** vorhandenen Raum bereits auszufüllen, so daß weniger Blut benötigt wird, um den gewünschten Druck aufzubauen. Dieser Effekt wird noch verstärkt, wenn als Füllstoff **106** ein Material verwendet wird, welches aufquillt, sobald es mit Blut in Berührung kommt. Als Füllstoff **106** wird vorzugsweise Mull, Watte, aufquellender Kunststoff oder dergleichen eingesetzt.

Der in **Figur 6** dargestellte Punktionsverschluß weist eine sehr große Aussparung **16** auf, in die ein am Verschlußelement **19** befestigtes Druckpolster **102** eingreift. Dabei weist das Druckpolster **102** ein nahezu inkompressibles Material auf.

Das Druckpolster **102** dient dazu, in der Druckkammer **10** ein gewisses Basisvolumen zu erzeugen. Dieses Basisvolumen wird dann durch das in die Druckkammer **10** einfließende Blut ergänzt und beschleunigt den Füllvorgang der Druckkammer **10**, so daß der Solldruck sehr viel schneller erreicht wird. Dadurch wird die benötigte Blutmenge deutlich verringert und eine Stillung der Blutung beschleunigt sowie der Blutverlust des Patienten verringert.

Ein derartiges Druckpolster **102** kann auch bei einem Punktionsverschluß gemäß den **Figuren 3** oder **4** eingesetzt werden.

In die Punktionsverschlüsse gemäß den **Figuren 1** bis **4** kann in die Druckkammer **10**, **30**, **40** alternativ oder zusätzlich zu dem Druckpolster **102** ein Füllstoff **106** vorgesehen sein:

Bei den vorgenannten Punktionsverschlüssen **1**, **3**, **4** ist sowohl das Halteband **11**, **31**, **41** als auch das Trägerelement **12** aus einem nahezu starren Material gefertigt, d.h., aus einem Material welches sich unter Druck oder Zug nicht nennenswert ausdehnt. Die Haltebänder **11**, **31**, **41** und das Trägerelement **12** können beispielweise aus einem Gewebeband, einem Pflaster, einem nicht nachgiebigem Kunststoffband oder dergleichen hergestellt sein. In den dargestellten Ausführungsformen sind die Haltebänder **11** und das Trägerelement **12** aus Inzisionsfolie gefertigt.

Die Druckwände **18**, **38**, **48** und der dehnbare Behälter **42** der vorgenannten Punktionsverschlüsse **1**, **3**, **4** sind aus einem sehr dehnbaren und/oder elastischen Material hergestellt, welches sich ohne großen Kraftaufwand dehnen bzw. vergrößern läßt. Derartige Materialen können Gummi, Latex, dehnbarer Kunststoff oder dergleichen sein.

Vor einem invasiven Eingriff nimmt der Arzt nun einen der oben beschriebenen Punktionsverschlüsse **1**, **3**, **4** und klebt in dort am Körper fest, wo der invasive Eingriff stattfinden wird. Dann sticht er die Kanüle **100** mit der daran befindlichen Spritze in die Arterie ein. Dadurch wird erreicht, daß der durch die Kanüle **100** erzeugte Einstichkanal mit der Öffnung **13**, **33**, **43** fluchtet.

Nachdem der invasive Eingriff des Arztes beendet ist, zieht dieser die Spritze zusammen mit der Kanüle **100** aus der Arterie und dem Punktionsverschluß **1**, **3**, **4** heraus und verschließt die Aussparung **16**, **36**, **46** in der Haltewand **17**, **37**, **47** des Punktionsverschlusses **1**, **3**, **4**, in dem er das vorbereitete Verschlußelement **19**, **39**, **49** über die Aussparung **16**, **36**, **46** klebt. Diese Klebeverbindung sollte ausreichend druckdicht sein, damit das nunmehr aus der Arterie durch den Einstichkanal in die Druckkammer **10**, **30**, **40** fließende Blut den zum Wundverschluß erforderlichen Druck in der Druckkammer **10**, **30**, **40** aufbauen kann, ohne daß diese leck wird.

Das aus der Arterie kommende Blut fließt nunmehr direkt in die Druckkammer **10**, **30**, **40** des Punktionsverschlusses **1**, **3**, **4** und nicht in das umliegende Gewebe, da der dem Blut entgegenwirkende Strömungswiderstand im Gewebe größer ist als im Punktionsverschluß **1**, **3**, **4**. Sobald die Druckkammer **10**, **30**, **40** gefüllt ist, baut sich hier ein Überdruck auf, der bewirkt, daß sich die Druckkammer **10**, **30**, **40** im Bereich der dehnbaren Druckwand **18**, **38**, **48** ausdehnt.

Eine Ausdehnung im Bereich der nahezu starren Haltewand **17**, **37**, **47** ist nicht möglich, da die Haltewand **17**, **37**, **47** nicht dehnbar ist und folglich nicht nachgibt.

Die sich ausdehnende Druckwand **18**, **38**, **48** drückt nun auf das Gewebe in der Nähe des Einstichkanales und erhöht somit den Druck im Gewebe. Hierdurch wird der Druck in der Druckkammer **10**, **30**, **40** und im Gewebe annähernd gleichzeitig aufgebaut, so daß das Blut stets in die Druckkammer **10**, **30**, **40** und nicht in das Gewebe fließt. Sobald der Druck in der Druckkammer **10**, **30**, **40** dem Blutdruck entspricht, stellt sich ein Gleichgewicht zwischen der Arterie und der Druckkammer **10**, **30**, **40** ein, so daß kein weiteres Blut in die Druckkammer **10**, **30**, **40** fließt.

Nun ist die Blutung zum Stillstand gekommen und das Blut kann gerinnen. Zur Beschleunigung der Gerinnung können in der Druckkammer Zusatzstoffe, wie z.B. Hämostatika oder dergleichen eingebracht sein.

Nachdem der Wundverschluß erfolgt ist, kann der Punktionsverschluß **1**, **3**,**4** vom Körper entfernt und fachgerecht entsorgt werden.

| **Bezugszeichenliste** | | | |
|---|---|---|---|
| 1 | 3 | 4 | Punktionsverschluß |
| 10 | 30 | 40 | Druckkammer |
| 11 | 31 | 41 | Halteband |
| | | 42 | Behälter |
| 12 | | | Trägerelement |
| 13 | 33 | 43 | Öffnung |
| 14 | 34 | 44 | Schutzfolie |
| 15 | 35 | 45 | Klebstoff |
| 16 | 36 | 46 | Aussparung |
| 17 | 37 | 47 | Haltewand |
| 18 | 38 | 48 | Druckwand |
| 19 | 39 | 49 | Verschlußelement |
| | | 100 | Kanüle |
| | | 102 | Druckpolster |
| | | 106 | Füllstoff |

## Patentansprüche

1. Punktionsverschluß zum Verschließen eines eine Punktionsöffnung aufweisenden Blutgefäßes in dem ein arterieller Druck herrscht, mit einer mit Überdruck beaufschlagbaren Druckkammer (10, 30, 40), die eine Öffnung (13, 33, 43) zur Aufnahme eines Druckmediums aufweist und die im Bereich der Punktionsöffnung am Körper befestigbar ist, wobei der dem Körper zugewandte Teil der Druckkammer (10, 30, 40) dehnbar ausgebildet ist, **dadurch gekennzeichnet**, daß die Öffnung (13, 33, 43) zur Aufnahme des Druckmediums derart in dem dem Körper zugewandten Teil der Druckkammer (10, 30, 40) angeordnet ist, daß die Öffnung (13, 33, 43) über einem Einstichkanal der Punktionsöffnung anbringbar ist und daß das aus der Punktionsöffnung des Blutgefäßes herausströmende Blut in die Druckkammer fließen kann, um dort das Druckmedium zu bilden.

2. Punktionsverschluß nach Anspruch 1, dadurch gekennzeichnet, daß der dem Körper zugewandte Teil der Druckkammer (10, 30, 40) als eine dehnbare Druckwand (18, 38, 48) ausgebildet ist und daß der dem Körper abgewandte Teil der Druckkammer (10, 30, 40) als eine nahezu nicht dehnbare haltewand (17, 37, 47) ausgebildet ist.

3. Punktionsverschluß nach Anspruch 2, dadurch gekennzeichnet, daß die nahezu nicht dehnbare Haltewand (37) durch ein Halteband (31) gebildet ist und daß die dehnbare Druckwand (38) druckdicht an dem Halteband (31) befestigt, vorzugsweise festgeklebt ist.

4. Punktionsverschluß nach Anspruch 2, dadurch gekennzeichnet, daß die dehnbare Druckwand (10) an einem am Halteband (11) befestigten Trägerelement (12) druckdicht befestigt, vorzugsweise festgeklebt ist.

5. Punktionsverschluß nach Anspruch 2, dadurch gekennzeichnet, daß die Druckkammer (40) aus einem dehnbaren, bis auf die Öffnung (43) zur Aufnahme des Druckmediums geschlossenen oder verschließbaren Behälter (42) gebildet ist.

6. Punktionsverschluß nach Anspruch 5, dadurch gekennzeichnet, daß der dehnbare Behälter (42) am Halteband (41) befestigt, vorzugweise festgeklebt ist.

7. Punktionsverschluß nach wenigstens einem der Ansprüche 1 bis 6, dadurch gekennzeichnet, daß die dehnbare Druckwand (38) oder der dehnbare Behälter (42) aus Gummi, Latex, Kunststoff oder dergleichen hergestellt ist.

8. Punktionsverschluß nach wenigstens einem der Ansprüche 3 bis 7, dadurch gekennzeichnet, daß das Halteband (11, 31, 41) luftdurchlässig ist.

9. Punktionsverschluß nach wenigstens einem der Ansprüche 3 bis 8, dadurch gekennzeichnet, daß das Halteband (11, 31, 41) auf der dem Körper zugewandten Seite zumindest teilweise mit Klebstoff (15, 35, 45) versehen ist.

10. Punktionsverschluß nach wenigstens einem der Ansprüche 1 bis 9, dadurch gekennzeichnet, daß in der Druckkammer (10) ein Blutgerinnungsmittel, ein Desinfektionsmittel, ein Hämostatika oder dergleichen angeordnet ist.

11. Punktionsverschluß nach wenigstens einem der Ansprüche 1 bis 10, dadurch gekennzeichnet, daß in der Druckkammer (10) ein Füllstoff (106) angeordnet ist.

12. Punktionsverschluß nach Anspruch 11, dadurch gekennzeichnet, daß der Füllstoff (106) aus einem Material hergestellt ist, welches bei Kontakt mit Blut aufquillt.

13. Punktionsverschluß nach wenigstens einem der Ansprüche 12 bis 13, dadurch gekennzeichnet, daß der Füllstoff (106) aus Mull, Watte, aufquellendem Kunststoff oder dergleichen hergestellt ist.

14. Punktionsverschluß nach wenigstens einem der Ansprüche 1 bis 13, dadurch gekennzeichnet, daß durch die Haltewand (17, 37, 47) und durch die Öffnung (13, 33, 43) zur Aufnahme eines Druckmediums hindurch eine Kanüle (100) angeordnet ist.

15. Punktionsverschluß nach Anspruch 14, dadurch gekennzeichnet, daß in der Haltewand (17, 37, 47) eine Aussparung zur Durchführung einer Kanüle vorgesehen ist, die verschließbar ist.

16. Punktionsverschluß nach Anspruch 15, dadurch gekennzeichnet, daß die Aussparung durch ein auf der Haltewand (17, 37, 47) aufgeklebtes Verschlußelement (19, 39, 49) verschließbar ist.

17. Punktionsverschluß nach Anspruch 16, dadurch gekennzeichnet, daß am Verschlußelement (19) ein Füllstoff (106) und/oder ein Druckpolster (102) angebracht ist.

## Claims

1. Puncture closure to close a puncture of a blood vessel being under arterial pressure, this puncture closure has a pressure chamber (10, 30, 40), which can be loaded with excess pressure, as well as an opening (13, 33, 43) for receiving the pressure medium and can be fastened onto the body in the vicinity of the puncture, whereas the body facing part of the pressure chamber (10, 30, 40) is extensible,
**characterized in that**
the opening (13, 33, 43) for receiving the pressure medium is arranged within the body facing part of the pressure chamber (10, 30, 40), so that the opening (13, 33, 43) can be located above a puncture channel of the puncture and that the blood flowing out of the puncture of the blood vessel itself can flow into the pressure chamber (10, 30, 40) for acting as pressure medium.

2. Puncture closure according to claim 1,
**characterized in that**
the body facing part of the pressure chamber (10, 30, 40) is designed as an extensible pressure wall (18, 38, 48) and that the part of the pressure chamber (10, 30, 40) being away of the body is designed as an almost not extensible retaining wall (17, 37, 47).

3. Puncture closure according to claim 2,
**characterized in that**
the almost not extensible retaining wall (37) is made of a retaining tape (31) and that the extensible pressure wall (38) is fastened, preferrably adhered in a pressure sealed way onto the retaining tape.

4. Puncture closure according to claim 2,
**characterized in that**
the extensible pressure wall (10) is fastened, preferrably adhered, in a pressure sealed way onto a carrier element (12) fastened onto the retaining tape (11).

5. Puncture closure according to claim 2,
**characterized in that**
the pressure chamber (40) is made of in an extensible receptacle (42), this receptacle (42) being closed or closable except for the opening (43) for receiving the pressure medium.

6. Puncture closure according to claim 5,
**characterized in that**
the extensible receptacle (42) is fastened, preferrably adhered onto the retaining tape (41).

7. Puncture closure according to at least one of the claims 1 to 6,
**characterized in that**
the extensible pressure wall (38) or the extensible receptacle (42) is made of rubber, latex, synthetic material or the like.

8. Puncture closure according to at least one of the claims 3 to 7,
**characterized in that**
the retaining tape (11, 31, 41) is breathable.

9. Puncture closure according to at least one of the claims 3 to 8,
**characterized in that**
the retaining tape (11, 31, 41) is at least partially provided with adhesive (15, 35, 45) on its body facing part.

10. Puncture closure according to at least one of the claims 1 to 9,
**characterized in that**
a coagulation agent, a disinfectant, a hemostatica or the like have are located in the pressure chamber (10).

11. Puncture closure according to at least one of the claims 1 to 10,
**characterized in that**
a filling agent (106) has been given into the pressure chamber (10).

12. Puncture closure according to claim 11,
**characterized in that**
the filling agent (106) is made of a material, which swells as soon as it gets in touch with blood.

13. Puncture closure according to at least one of the claims 11 to 12,
**characterized in that**
the filling agent (106) is made of gauze, cotton, swelling synthetic material or the like.

14. Puncture closure according to at least one of the claims 1 to 13,
**characterized in that**
a cannula (100) has been inserted through the retaining wall (17, 37, 47) and through the opening (13, 33, 43) for receiving a pressure medium.

15. Puncture closure according to claim 14,
**characterized in that**
an opening has been provided in the retaining wall (17, 37, 47) through which a cannula can be inserted.

16. Puncture closure according to claim 15,
**characterized in that**
the opening can be closed by means of a closing element (19, 39, 49) adhered onto the retaining wall (17, 37, 47).

17. Puncture closure according to claim 16,
**characterized in that**
a filling agent (106) and/or a pressure pad (102) is connected to the closing element (19).

## Revendications

1. Fermeture de ponction pour fermer un vaisseau sanguin ponctionné dans lequel règne une tension artérielle; cette fermeture a une chambre sous pression (10, 30, 40) pouvant être surpressurisée et un orifice (13, 33, 43) pour recevoir le produit de pression, et peut être fixée au corps à l'endroit de la ponction, la partie de la chambre sous pression (10, 30, 40) faisant face au corps étant extensible,
**caractérisée en ce que** l'orifice (13, 33, 43) recevant le produit de pression est placé dans la partie de la chambre sous pression (10, 30, 40) faisant face au corps de telle sorte que l'orifice (13, 33, 43) puisse être placé par-dessus un canal de piqûre de la ponction et que le sang s'écoulant du vaisseau sanguin ponctionné puisse couler dans la chambre sous pression pour y faire fonction de produit de pression.

2. Fermeture de ponction selon la revendication 1,
**caractérisée en ce que** la partie de la chambre sous pression (10, 30, 40) faisant face au corps est conçue comme une paroi de pression extensible (18, 38, 48) et que la partie de la chambre sous pression (10, 30, 40) se trouvant loin du corps est conçue comme une paroi de retenue (17, 37, 47) à peine extensible.

3. Fermeture de ponction selon la revendication 2,
**caractérisée en ce que** la paroi de retenue à peine extensible (37) est formée par une bande de retenue (31) et que la paroi de pression extensible (38) est fixée à la bande de retenue (31) de façon à être étanche sous pression, de préférence en l'y collant.

4. Fermeture de ponction selon la revendication 2,
**caractérisée en ce que** la paroi de pression extensible (10) est fixée à un élément de support (12) attaché à la bande de retenue (11) de façon a être étanche sous pression, de préférence en l'y collant.

5. Fermeture de ponction selon la revendication 2,
**caractérisée en ce que** la chambre sous pression (40) est faite d'un récipient (42) extensible fermé ou fermant à l'exception de l'orifice (43) recevant le produit de pression.

6. Fermeture de ponction selon la revendication 5,
**caractérisée en ce que** le récipient extensible (42) est fixé à la bande de retenue (41), de préférence en l'y collant.

7. Fermeture de ponction selon au moins une des revendications 1 à 6,
**caractérisée en ce que** la paroi de pression extensible (38) ou le récipient extensible (42) est fait en caoutchouc, latex, plastique ou autre matériel similaire.

8. Fermeture de ponction selon au moins une des revendications 3 à 7,
**caractérisée en ce que** la bande de retenue (11, 31, 41) est perméable à l'air.

9. Fermeture de ponction selon au moins une des revendications 3 à 8,
**caractérisée en ce que** la bande de retenue (11, 31, 41) est pourvue, du moins en partie, de colle (15, 35, 45) sur le côté faisant face au corps.

10. Fermeture de ponction selon au moins une des revendications 1 à 9,
**caractérisée en ce qu'** un coagulant, un désinfectant, un hémostatique ou autre produit similaire est mis dans la chambre sous pression (10).

11. Fermeture de ponction selon au moins une des revendications 1 à 10,
**caractérisée en ce qu'** un agent de remplissage (106) est mis dans la chambre sous pression (10).

12. Fermeture de ponction selon la revendication 11,
**caractérisée en ce que** l'agent de remplissage (106) est fait d'un matériel qui gonfle au contact du sang.

13. Fermeture de ponction selon la revendication 12 à 13,
**caractérisée en ce que** l'agent de remplissage (106) est fait de gaze, d'ouate, de plastique gonflant ou d'un autre matériel similaire.

14. Fermeture de ponction selon la revendication 1 à 13,
**caractérisée en ce qu'** une canule (100) est insérée au travers de la paroi de retenue (17, 37, 47) et de l'orifice (13, 33, 43) pour recevoir un produit de pression.

15. Fermeture de ponction selon la revendication 14,
**caractérisée en ce que** la paroi de retenue (17, 37, 47) est pourvue d'une ouverture fermante permettant le passage d'une canule.

16. Fermeture de ponction selon la revendication 15,
**caractérisée en ce que** l'ouverture peut être fermée par un élément de fermeture (19, 39, 49) collé sur la paroi de retenue (17, 37, 47).

17. Fermeture de ponction selon la revendication 16,
**caractérisée en ce qu'** un agent de remplissage (106) et/ou un tampon (102) sont attachés sur l'élément de fermeture.
